# EUROPEAN PATENT APPLICATION

(11) **EP 4 442 823 A1**
(43) Date of publication of application: **09.10.2024**
(21) Application number: 22911447.5
(22) Date of filing: 27.01.2022
(51) Int. Cl.: C12N 15/10, C12Q 1/6806, C12Q 1/686

(54) **NUCLEIC ACID EXTRACTION METHOD USING POROUS ION CHARGE CRYSTAL**

(30) Priority: 23.12.2021 KR 20210185773
(71) Applicant: Neuclacid Inc., Seoul 08502 (KR)
(72) Inventor: HONG, Seungbum, Namyangju-si Gyeonggi-do 12158 (KR); JUNG, SoonIm, Ulju-gun Ulsan 44953 (KR); HA, Myong Gi, Seoul 03723 (KR)
(74) Representative: BCKIP Part mbB
(86) International application number: PCT/KR2022/001489
(87) International publication number: WO 2023/120807

(57) **Abstract**

The present invention relates to porous ion charge particles for nucleic acid isolation and a method of extracting nucleic acids using the same. More specifically, the porous ion charge particles of the present invention have anionic properties, so that they may electrically isolate negatively charged nucleic acids, adsorb cationic by-products to the particles, and simultaneously absorb water, which is a solvent. In other words, the isolation and concentration of nucleic acids are automatically carried out simply by immersing the porous ion charge particles in a reaction tube containing a biological sample, so there is an effect that nucleic acids can be simply and quickly isolated and concentrated without using harmful organic substances, magnetic beads, or complex devices such as centrifuge.

## Description

### TECHNICAL FIELD

The present invention relates to porous ion charge particles and a method for nucleic acid extraction using the same.

### BACKGROUND ART

Currently, technologies for extracting nucleic acids from biological samples such as cells, bacteria, or viruses are widely used in connection with nucleic acid amplification reaction technology for basic research in the field of molecular biology and for diagnosis, treatment, or prevention of diseases. In addition, technologies for extracting nucleic acids from biological samples are required in various fields such as customized new drug development, forensic medicine, and environmental hormone detection.

As an example of a conventional nucleic acid extraction technology, there is a method of purifying nucleic acids by treating a sample containing cells with sodium dodecyl sulfate (SDS) or proteinase K to solubilize it, and then denaturing and decomposing proteins using phenol/chloroform or phenol/chloroform/isoamyl alcohol, and removing by-products through ethanol precipitation. However, the phenol extraction method has problems that the method not only takes a lot of time because it requires many processing steps, but also has a significantly low reliability because the nucleic acid extraction efficiency is greatly influenced by the researcher's experiences and skills. Furthermore, the conventional nucleic acid extraction methods have the problem that histone proteins or the like remain as by-products during the cell disruption process, which is essential for cell lysis, and act as inhibitors that interfere with polymerase chain reaction (PCR).

Recently, kits using silica or glass fiber that specifically binds to nucleic acids have been used to solve these problems. Since the silica or glass fiber has a low binding ratio with proteins and cell metabolites, a relatively high concentration of nucleic acids may be obtained. This method has the advantage of being simple compared to the phenol method, but since it uses a chaotropic reagent or ethanol that strongly inhibits enzymatic reactions such as PCR, the method has the disadvantage that these substances must be completely removed, and for this reason, the manipulation is cumbersome and takes a lot of time.

Recently, a method for directly purifying nucleic acids using a filter was disclosed in International Patent Publication No. 2000/21973. This method involves passing a sample through a filter, adsorbing cells to the filter, lysing the cells adsorbed on the filter, filtering the cells with a filter, and then washing and eluting nucleic acids adsorbed on the filter.

For example, Korea Patent Registration No. 10-0454869 discloses a DNA extraction method using a cell lysis buffer, and the conventional nucleic acid extraction method includes steps of 1) adding a cell lysis buffer to cells to lyse the cells; 2) transferring the lysed cells from step 1 to a filter and fixing nucleic acids; 3) washing the filter of step 2; and 4) recovering the nucleic acids from the filter, so it has the advantage of allowing for the extraction of nucleic acids with high reproducibility in a few steps and within a short time. However, since the use of a centrifuge is essential when transferring lyse cells to a filter, washing the filter, or recovering nucleic acids from the filter, the method has low portability and mobility and complicates the in-situ nucleic acid extraction.

There are also a nucleic acid extraction method using magnetic beads, a nucleic acid extraction method using syringes and filters, and a nucleic acid extraction method using Trizol. However, the nucleic acid extraction method using magnetic beads requires a magnet to fix nucleic acids to the wall of a tube, and removing the solution requires the use of a pump or valve (automated device) or multiple tips and pipettes (manual device). Furthermore, there is a problem that some nucleic acids may be lost during the nucleic acid washing and elution processes. The nucleic acid extraction method using syringes and filters has the problem that when a force exceeding a certain strength is applied during the process of transferring nucleic acids using a syringe, the filter may be damaged, making extraction of the nucleic acid difficult. In addition, the nucleic acid extraction method using Trizol has the problem of using harmful organic solvents such as phenol or chloroform.

Therefore, there is a need to develop a nucleic acid extraction method that can solve the problems of the conventional nucleic acid extraction methods and easily and efficiently isolate nucleic acids at high concentrations.

### DETAILED DESCRIPTION OF THE INVENTION TECHNICAL PROBLEM

The present invention is intended to solve the problems of the conventional technologies, and a main object of the present invention is to provide porous ion charge particles for nucleic acid extraction and a method for nucleic acid extraction using the same, which may improve user convenience and allow for quickly performing in-situ reactions by minimizing the use of pipettes and tips.

In addition, another object of the present invention is to provide porous ion charge particles for nucleic acid extraction and a method for nucleic acid extraction using the same, which have excellent portability and mobility because porous ion charge particles that can utilize the electrical properties of nucleic acids are used and so the use of harmful organic solvents, magnetic substances, or complicated instruments or devices is not required.

In addition, still another object of the present invention is to provide porous ion charge particles for nucleic acid extraction and a method for nucleic acid extraction using the same, which use porous ion charge particles to adsorb cationic by-products to the particles and absorb water, thereby isolating and concentrate only pure nucleic acids.

### TECHNICAL SOLUTION

As a means to achieve the above-described objects of the present invention, the present invention provides porous ion charge particles for nucleic acid isolation.

In addition, the present invention provides a nucleic acid extraction tube including the porous ion charge particles for nucleic acid isolation.

In addition, the present invention provides a method of extracting nucleic acids, including: adding and mixing a sample including nucleic acids into a nucleic acid extraction tube together with a direct lysis buffer; adding porous ion charge particles to the resulting mixture and subjecting to a reaction for 5 to 30 minutes; and removing the ion charge particles that have completed the reaction and obtaining a buffer solution in which nucleic acids are concentrated.

In addition, the present invention provides a method of extracting nucleic acids, including: adding and mixing a sample including nucleic acids into a nucleic acid extraction tube together with a direct lysis buffer; adding porous ion charge particles to the resulting mixture and subjecting to a reaction for 5 to 30 minutes; removing the ion charge particles that have completed the reaction and obtaining a buffer solution in which nucleic acids are concentrated; adding a polymerase chain reaction (PCR) mixed agent to the buffer solution in which nucleic acids are concentrated and performing a PCR; and in-situ detecting a sample during the PCR or after completing the PCR.

### ADVANTAGEOUS EFFECTS

According to the porous ion charge particles for nucleic acid isolation and the method of extracting nucleic acids using the same according to the present invention, the isolation and concentration of nucleic acids are automatically carried out simply by immersing the porous ion charge particles in a reaction tube containing a biological sample, so there is an effect that nucleic acids can be simply and quickly isolated and concentrated without using harmful organic substances, magnetic beads, or complex devices such as centrifuge.

In addition, the present invention can be effectively applied to samples containing low-concentration biomaterials (pathogens, etc.) because lysis, purification, and concentration are carried out directly from a small amount of biological sample without pretreatment, and therefore, the present invention can be widely used in various fields such as various basic studies, which have been limited by low concentrations, and diagnostic testing, clinical diagnosis, environmental monitoring, and pharmacogenetic analysis in which the sample volume is extremely limited.

### DESCRIPTION OF THE DRAWINGS

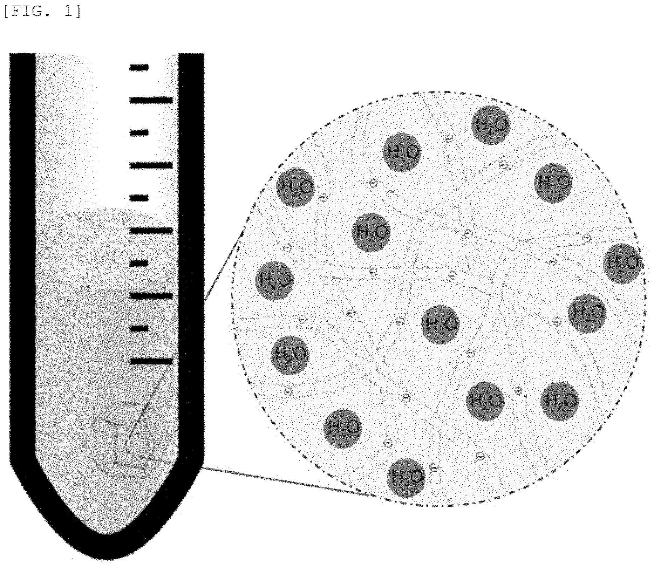

FIG. 1 shows an enlarged diagram illustrating a porous structure of the ion charge particles of the present invention.

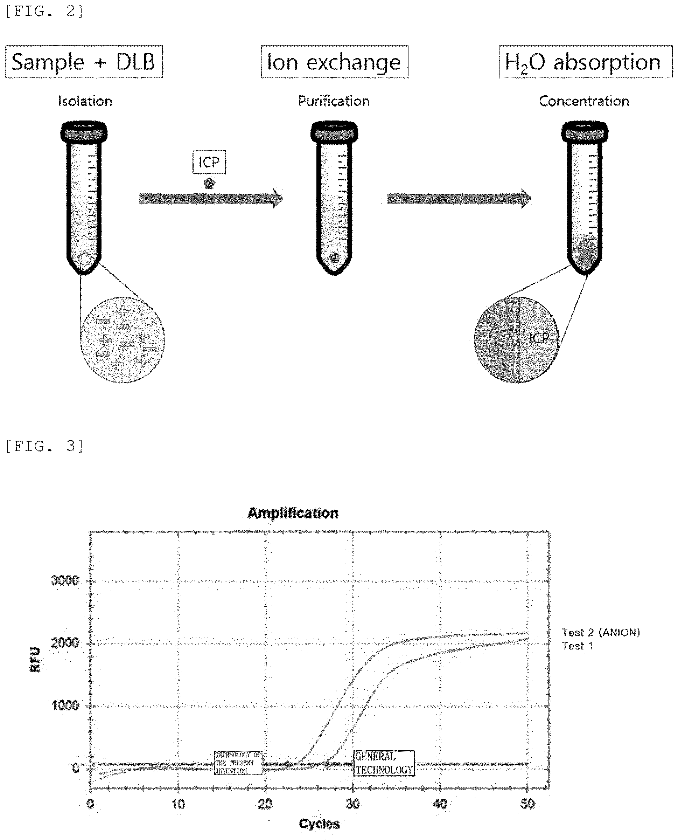

FIG. 2 shows a schematic diagram briefly illustrating processes of the method of extracting nucleic acids of the present invention. The diagram shows processes in which a biological sample is lysed by a dissolution buffer, nucleic acids and by-products are separated, and the ion charge particles attach cationic by-products and simultaneously absorb water to obtain a buffer solution in which nucleic acids are concentrated.

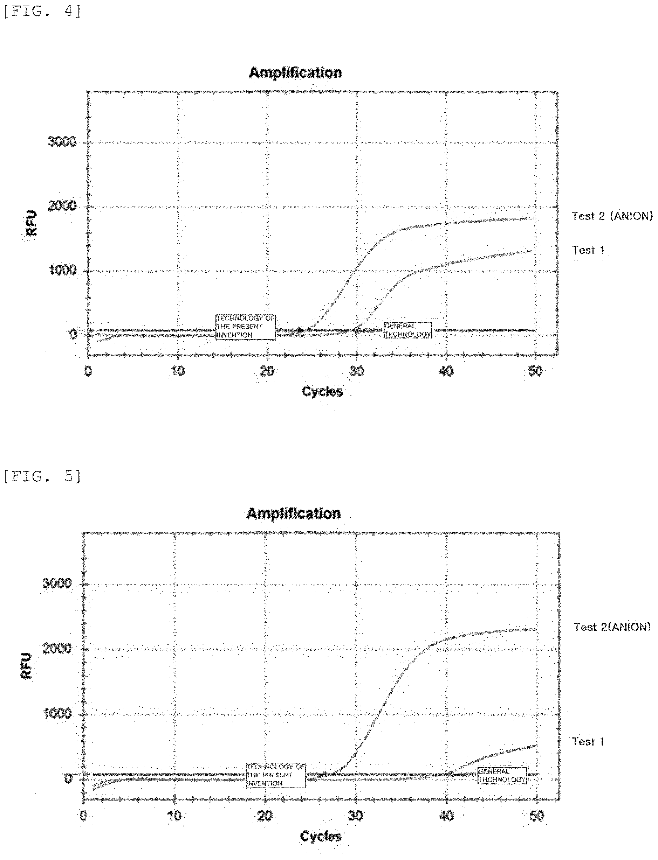

FIGS. 3 and 4 show the results of comparing the real-time polymerase chain reaction (PCR) amplification efficiency of a target gene (glyceraldehyde-3-phosphate dehydrogenase, GAPDH) after applying each of the method of extracting nucleic acids of the present invention (Test 2) and an extraction method using only a direct lysis buffer (DLB) to an oropharyngeal sample (oropharyngeal cancer sample).

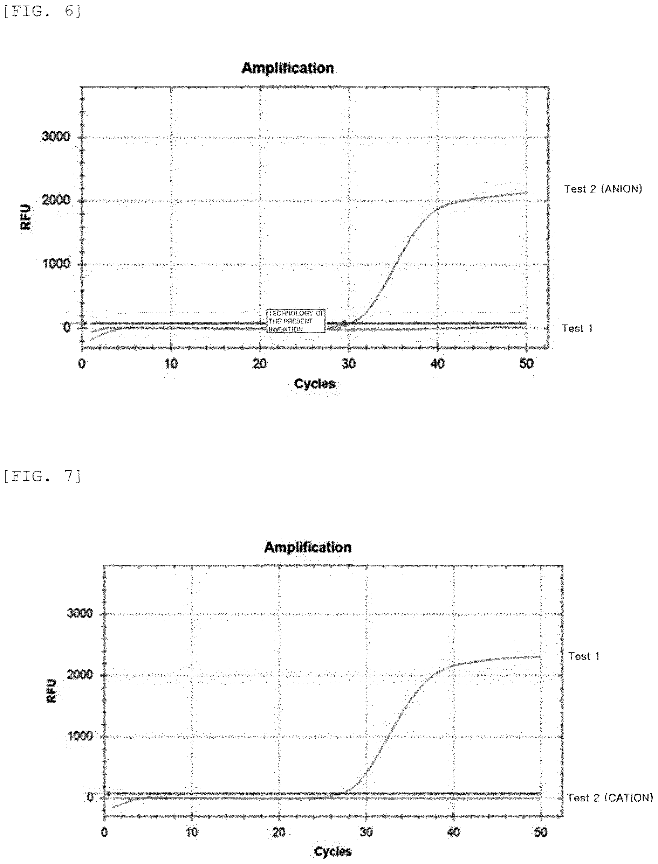

FIGS. 5 and 6 show the results of comparing the real-time PCR amplification efficiency of a target gene (GAPDH) after applying each of the method of extracting nucleic acids of the present invention (Test 2) and an extraction method using only a DLB to a nasopharyngeal sample (nasopharyngeal cancer sample).

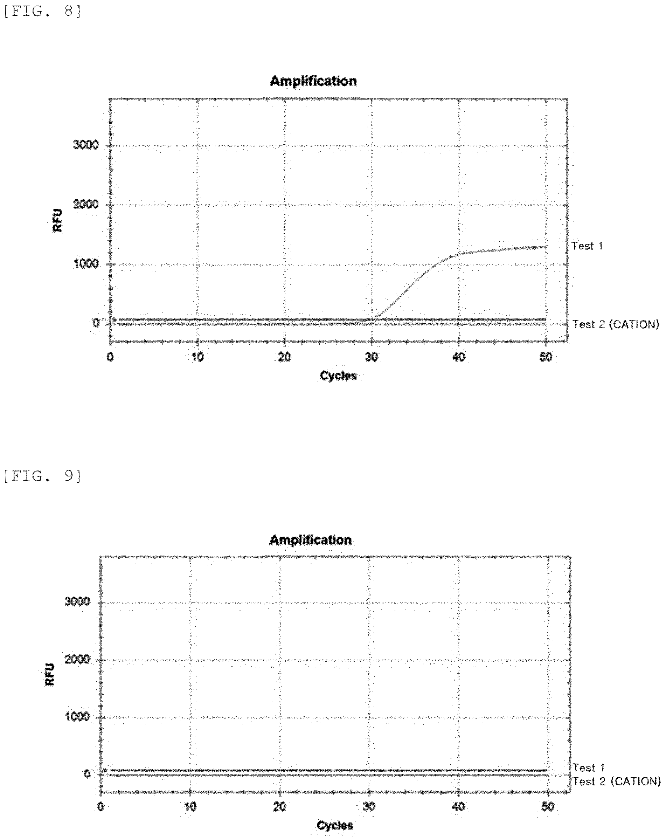

FIGS. 7 and 8 show the results of comparing the real-time PCR amplification efficiency of a target gene (GAPDH) after applying each of the extraction method using the cation charge particles of Example 1 below (Test 2) and an extraction method using only a DLB to an oropharyngeal sample (oropharyngeal cancer sample).

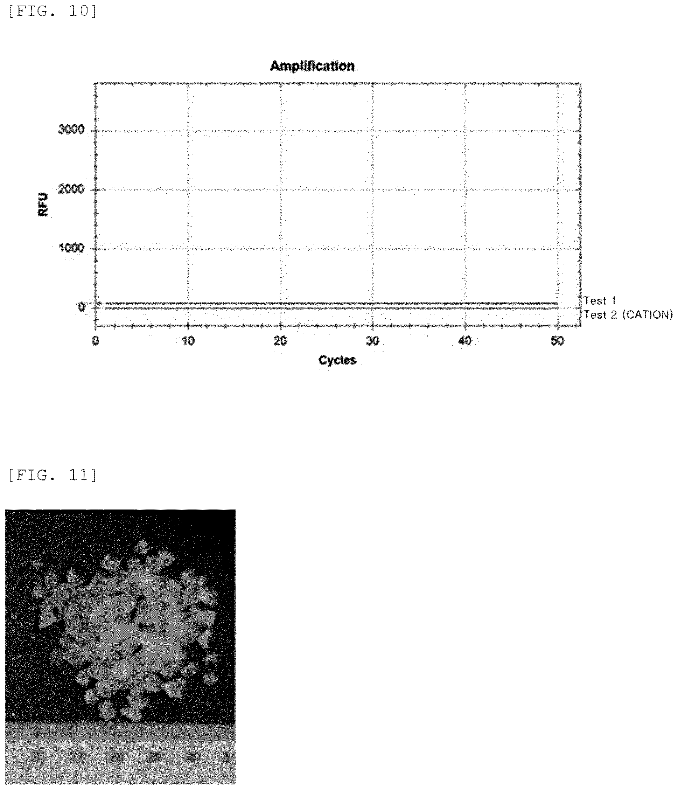

FIGS. 9 and 10 show the results of comparing the real-time PCR amplification efficiency of a target gene (GAPDH) after applying each of the extraction method using the cation charge particles of Example 2 below (Test 2) and an extraction method using only a DLB to a nasopharyngeal sample (nasopharyngeal cancer sample).

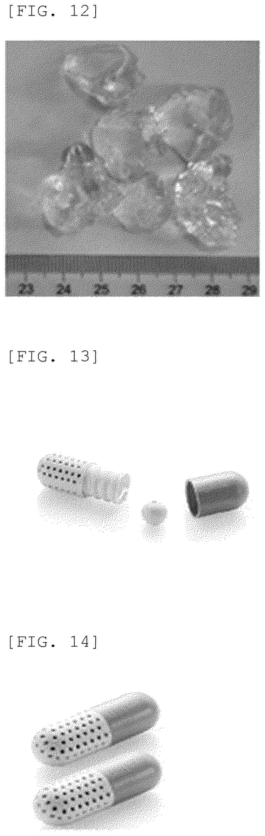

FIGS. 11 and 12 show photographs comparing the sizes of the porous ion charge particles before and after water absorption.
FIGS. 13 to 15 show conceptual images illustrating the external appearance of a capsule containing ion charge particles.

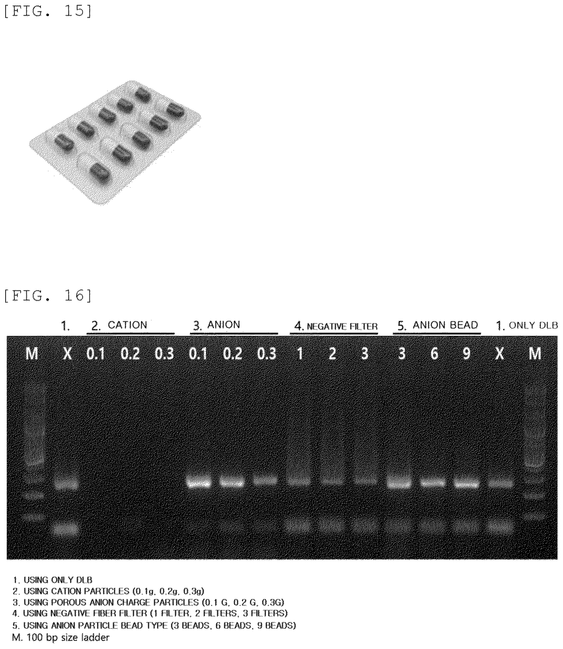

FIG. 16 shows the results of confirming the nucleic acid isolation efficacy of each method through PCR detection tests after isolating and concentrating nucleic acids using various types of polymer absorbers.

### BEST MODE

The present invention will be explained and made clear through embodiments described in detail below in conjunction with the accompanying drawings. Terms or words used in describing the present invention should not be construed as limited to their conventional or dictionary meanings, but should be construed as having a meaning and concept consistent with the technical idea of the present invention based on the principle that the inventor may appropriately define the concept of terms in order to explain their invention in the best way.

In addition, the embodiments described to explain the present invention and the configurations illustrated in the drawings are only the most preferred embodiments of the present invention and do not encompass the entire technical idea of the present invention, so there may be equivalent modification examples that may replace them at the time of filing the present application.

In addition, in describing the present invention, when a detailed description of a related known configuration or function is considered as obscuring the gist of the present invention, the detailed description will be omitted.

Terms used in the present specification may be defined as described below.

"Porous ion charge particles" refer to electrically charged particles with many small holes (pores) formed on the surface and inside of a solid.

"Sample" is not limited to certain types or forms as long as they may contain biological materials to be detected. For example, a sample may be a biological sample, for example, a biological fluid or a biological tissue. Examples of biological fluid may include urine, blood, plasma, serum, saliva, semen, feces, sputum, cerebrospinal fluid, tears, mucus, and amniotic fluid. A biological tissue is a collection of cells, generally, a collection of intracellular substances forming one of the structural materials of human, animal, plant, bacterial, fungi or viral structures with specific types, and it may be a connective tissue, an epithelial tissue, a muscular tissue, and a nervous tissue. In addition, examples of biological tissues may also include organs, tumors, lymph nodes, arteries, and individual cell(s). In addition, samples may include environmental samples containing biological substances at low concentrations and may include a variety of forms and types, for example, drinking water, food, and the like.

"Nucleic acid" may refer to a plurality of nucleotides consisting of a sugar linked to an organic base (a substituted pyrimidine such as cytosine, thymidine or uracil or a substituted purine such as adenine or guanine). In other words, examples include ribonucleic acid (RNA), deoxyribonucleic acid (DNA), methyl phosphonate nucleic acid, S-oligo, c-DNA, cRNA, miRNA, siRNA, aptamer, and the like, and it may be a concept encompassing anything that occurs naturally or that is artificially synthesized. However, more typically, it may be DNA, RNA, and the like.

"Direct lysis buffer (DLB)" is a buffer used when destroying cells, and it is a composition containing a salt or/and a surfactant to adjust the acidity and osmotic pressure of a lysate. For example, it may refer to a composition that can effectively maintain a pKₐ of about 5.8 to about 9 at 25 °C and a pH value of, for example, 6 to 9 (specifically, about 7.5 to 9, more specifically, about 7.8 to 8.5).

"Cell lysis or lysis" refers to a phenomenon in which the cell membrane ruptures and the cell contents are exposed at the same time due to the decomposition of cells or the like, and generally, it is often used to isolate DNA or RNA at all stages of an amplification process such as PCR.

"PCR" refers to a reaction in which a large amount of identical DNA strands are formed from one initial template by a cycling process (heating and cooling performed alternately). Generally, a PCR mixture may include (i) a double-helical DNA molecule, which is a template having a base sequence to be amplified, (ii) a primer (a single-stranded DNA molecule capable of binding to a complementary DNA base sequence in the template DNA), (iii) a mixture of dNTPs, which are dATP, dTTP, dGTP, and dCTP (nucleotide subunits that combine to form a new DNA molecule during the PCR amplification process), and (iv) a Taq DNA polymerase (an enzyme that synthesizes a new DNA molecule using dNTPs) .

The present invention provides porous ion charge particles for nucleic acid isolation.

According to an embodiment of the present invention, the ion charge particles may be anionic particles having a functional group or a moiety exhibiting a negative charge, and any type of particles having a negative charge may be used without limitation.

Since nucleic acids have a negative charge characteristic due to its structure, they are electrically repelled by the anion charge particles of the present invention and thus are unable to bind to the ion charge particles, while a lysis by-product with a positive charge characteristic binds to the ion charge particles.

In addition, according to another embodiment of the present invention, the ion charge particles have a superporous structure and may absorb a solvent, especially water (H₂O).

The superporous structure provides a surface area hundreds of times larger than that of a typical porous structure, thereby providing a very short diffusion distance. In addition, the pores inside the ion charge particles may have a structure in which the pores are connected to each other, and water may be absorbed at a very high rate due to an capillary action, and the water absorption and swelling rate of the porous ion charge particles of the present invention may be hundreds of times higher than that of a typical porous particles.

According to one embodiment of the present invention, a component of the "porous ion charge particles" may be used without limitation as long as it is a material that is porous and has a negative charge, and it may be a synthetic resin or natural resin.

The "porous ion charge particles" of the present invention may be an anionic polymer, and specifically, they may be a polyelectrolyte that has a dissociation group in the polymer chain and dissociates when in contact with water to form polymer ions.

According to one embodiment of the present invention, a component of the "porous ion charge particles" may be one or more selected from polyacrylate, sodium polyacrylate, polyacrylamide, polyvinyl alcohol, polyethylene, polyacrylonitrile, alginate, ethylene maleic anhydride, methyl cellulose, hydroxy ethyl cellulose, hydroxypropyl methyl cellulose, cellulose acetate, carboxy methyl cellulose, polymethacrylate (PMA), and the like, and preferably, it may be sodium polyacrylate. Of course, it is not limited thereto.

More specifically, the porous ion particles of the present invention have a three-dimensional network structure in which polyelectrolyte materials are cross-linked with each other. The osmotic pressure and three-dimensional network structure of these polymer electrolytes allow for absorption and storage of a solution.

According to one embodiment of the present invention, the porous ion particles may be a polymer composed of acrylic acid, and may be prepared by forming a large lump according to gel polymerization and then cutting it to fit a desired particle size. Therefore, the shape of the particles is irregular and amorphous, and through subsequent processing, the particles may be classified and prepared into desired particle shape and size. In addition, the absorption power of porous ion particles varies depending on the ionic properties of an electrolyte in contact, and when anions (SO₄²-, OH-, etc.) are dissolved in pore water and an alkaline environment is created, an anionic polymer material is prepared.

An acrylic acid-based polymer used in the present invention may include an anionic functional group such as a carboxyl group (-COO⁻) in an anionic electrolyte environment.

The porous ion charge particles may have various shapes such as amorphous, spherical, elliptical, and polygonal shapes. According to one embodiment of the present invention, the porous ion charge particles may have an average diameter of about 0.05 to 1.5 cm before solvent absorption, preferably 0.1 to 1.0 cm, 0.2 to 0.8 cm, and most preferably 0.3 to 0.5 cm.

According to another embodiment of the present invention, the volume of the porous ion charge particles may increase by 1,000 to 50,000% (10 to 500 folds) after absorbing a solution compared to that before solvent absorption, preferably 20,000 to 30,000% (200 to 300 folds).

As an example of the present invention, the pore size (diameter) of the porous ion charge particles may be in the range, for example, from about 0.1 to 10 nm, specifically from about 1 to 5 nm, and more specifically from about 3 to 5 nm. When the pore size is too large, the ability to absorb water may be reduced, and when the pore size is small, the ability to absorb by-products may be reduced. In consideration of this, it may be preferable to use porous ion charge particles with an appropriate pore size.

In addition, the present invention may provide a nucleic acid extraction tube including the porous ion charge particles for nucleic acid isolation.

The nucleic acid extraction tube is a structure for measuring nucleic acids in a sample containing nucleic acids, and may include a body portion that can accommodate a sample, a cap that opens and closes an opening of a certain body portion, and a connecting portion between the body portion and the cap, but is limited thereto, and it may also include a form in which the body portion and the cap are not connected by a connecting portion but are present separately.

A material forming the nucleic acid extraction tube may be at least one of polypropylene (PP), polyethylene (PE), polyvinyl chloride (PVC), polycarbonate (PC), polyester, polymethyl methacrylate (PMMA), polyethylene terephthalate (PET), polyurethane (PU), silicon, glass, ceramic, and Teflon, but is not limited thereto.

At this time, when moisture absorption by plastic is not a problem, preferred plastics may include acrylonitrile butadiene styrene (ABS), acetal, acrylic fibers, acrylonitrile, cellulose acetate, ethyl cellulose, alkylvinylalcohol, polyaryletherketone, polyetheretherketone, polyetherketone, melamine formaldehyde, phenolic formaldehyde, polyamide (e.g. nylon 6, nylon 66, nylon 12), polyamide-imide, polydicyclopentadiene, polyether-imide, polyethersulfone, polyimide, polyphenyleneoxide, polyphthalamide, methylmethacrylate, polyurethan, polysulfone, and vinyl formal, but are not limited thereto.

In addition, when moisture absorption by plastic is a problem, preferred plastics may include polystyrene, polypropylene, polybutadiene, polybutylene, epoxy, Teflon, peroxyacetylnitrate (PAN), polyethylene terephthalate (PET), polytetrafluoroethylene (PTFE), chloro-fluoroethylene, polyvinylidene fluoride, liquid crystal polymers, polyester, low-density polyethylene (LDPE), high-density polyethylene (HDPE), polymethylpentene, polyphenylene sulfide, polyolefin, polyvinyl chloride (PVC), and chlorinated PVC, but are not limited thereto.

The sample volume that the nucleic acid extraction tube may accommodate may be in the range from 1 to 50,000 µl, but is not limited thereto, and the tube may be formed in various sizes depending on a user's purpose and so the volume may have a wider range.

The body portion of the nucleic acid extraction tube may be formed in various shapes. Depending on a user's purpose, the shape of the lower portion may be formed in various shapes such as an arc shape or a rectangular shape.

Therefore, the nucleic acid extraction tube according to one embodiment of the present invention may include a body portion having various shapes, volumes, and shapes, thereby providing a tube suitable for a user's purpose.

In addition, the present invention may provide a method of extracting nucleic acids, including:
adding and mixing a sample including nucleic acids into a nucleic acid extraction tube together with a direct lysis buffer (DLB);
adding porous ion charge particles to the resulting mixture and subjecting to a reaction for 5 to 30 minutes; and
removing the ion charge particles that have completed the reaction and obtaining a buffer solution in which nucleic acids are concentrated.

According to one embodiment of the present invention, the porous ion charge particles are anionic, and anionic ion charge particles may adsorb cationic by-products. In addition, the porous ion charge particles of the present invention absorb a solvent, especially water.

The physical and chemical properties of the materials and particles that may be used as the porous ion charge particles are the same as described above.

According to one embodiment of the present invention, any type of DLB may be used without particular limitations as long as it is a buffer solution that removes cell membranes and nuclear membranes and elutes nucleic acids, and the type of salt used as necessary and the type, concentration, pH, and the like of a surfactant may be adjusted.

The amount of porous ion particles added to the mixture may be appropriately adjusted depending on the type and amount of the sample. According to one example of the present invention, 10 to 500 mg may be added, preferably 10 to 400 mg, 50 to 350 mg, or 100 to 300 mg may be added.

According to one embodiment of the present invention, the reaction may be performed for 5 minutes to 20 minutes, for example, 5 minutes to 15 minutes, 5 minutes to 10 minutes, or 10 minutes to 15 minutes, and it may be performed, for example, about 10 minutes.

According to an embodiment of the present invention, the nucleic acid extraction reaction may be performed at room temperature and may be a nucleic acid extraction method including no further washing.

In addition, the present invention may further include isolating nucleic acids from a buffer solution after removing ion charge particles and obtaining a buffer solution in which nucleic acids are concentrated.

According to another embodiment of the present invention, the present invention may provide a method of extracting nucleic acids, including:
adding and mixing a sample including nucleic acids into a nucleic acid extraction tube together with a direct lysis buffer;
adding porous ion charge particles to the resulting mixture and subjecting to a reaction for 5 to 30 minutes;
removing the ion charge particles that have completed the reaction and obtaining a buffer solution in which nucleic acids are concentrated;
adding a polymerase chain reaction (PCR) mixed agent to the buffer solution in which nucleic acids are concentrated and performing a PCR; and
in-situ detecting a sample during the PCR or after completing the same.

### Examples

### Preparation Example 1: Preparation of porous ion charge particles

In the examples of the present invention, sodium polyacrylate (LG Household & Health Care Co., Ltd.) was used as an anionic polymer, and in the Comparative Examples, styrenedivinylbenzene copolymers (DOW Inc.) were used as cationic polymers.

### Example 1: Verification of nucleic acid detection effect in oropharyngeal sample (target: GAPDH)

The results (Test 2) of performing RT-PCR after applying the nucleic acid extraction method (Test 2; anion) using ion charge particles according to an embodiment of the present invention to an oropharyngeal sample (oropharyngeal cancer sample) and the results (control, Test 1) of performing RT-PCR after applying only the DLB to the same sample of the same amount were compared and illustrated using fluorescence amplification curves, and the results are shown in FIGS. 3 and 4.

As shown in the graph of FIG. 3, in the case of Test 1, fluorescence began to appear from 26 cycles, and the relative fluorescence unit (RFU) at the end point was found to have a level within the range of about 2000, and in the case of Test 2 (anion), fluorescence began to appear from 23 cycles, and the RFU at the end point was found to have a level within the range of about 2200. As shown in the graph of FIG. 4, in the case of Test 1, fluorescence began to appear from 29 cycles, and the RFU at the end point was found to have a level within the range of about 1300, and in the case of Test 2 (anion), fluorescence began to appear from 24 cycles, and the RFU at the end point was found to have a level within the range of about 1900.

### Example 2: Verification of nucleic acid detection effect in nasopharyngeal sample (target: GAPDH)

The results (Test 2) of performing RT-PCR after applying the nucleic acid extraction method (Test 2; anion) using ion charge particles according to an embodiment of the present invention to a nasopharyngeal sample (nasopharyngeal cancer sample) and the results (control, Test 1) of performing RT-PCR after applying only the DLB to the same sample of the same amount were compared and illustrated using fluorescence amplification curves, and the results are shown in FIGS. 5 and 6.

As shown in the graph of FIG. 5, in the case of Test 1, fluorescence began to appear from 30 cycles, and the RFU at the end point was found to have a level within the range of about 600, and in the case of Test 2 (anion), fluorescence began to appear from 27 cycles, and the RFU at the end point was found to have a level within the range of about 2300. As shown in the graph of FIG. 6, in the case of Test 1, no fluorescence appeared, and in the case of Test 2 (anion), fluorescence began to appear from 30 cycles, and the RFU at the end point was found to have a level within the range of about 2100.

According to the results of Examples 1 and 2 above, it was found that the nucleic acid extraction method using the porous ion charge particles of the present invention have superior nucleic acid extraction and detection effects than the nucleic acid extraction method using only the DLB.

### Comparative Example 1: Verification of nucleic acid detection effect in oropharyngeal sample (target: GAPDH) using cation charge particles

The results (Test 2) of performing RT-PCR after applying the DLB and the nucleic acid extraction method (Test 2; cation) using cation charge particles to an oropharyngeal sample (nasopharyngeal cancer sample) and the results (control, Test 1) of performing RT-PCR after applying only the DLB to the same sample of the same amount were compared and illustrated using fluorescence amplification curves, and the results are shown in FIGS. 7 and 8.

As shown in the graph of FIG. 7, in the case of Test 1, fluorescence began to appear from 27 cycles, and the RFU at the end point was found to have a level within the range of about 2300, and in the case of Test 2 (cation), no fluorescence appeared. As shown in the graph of FIG. 8, in the case of Test 1, fluorescence began to appear from 29 cycles, and the RFU at the end point was found to have a level within the range of about 1300, and in the case of Test 2 (cation), no fluorescence appeared.

### Comparative Example 2: Verification of nucleic acid detection effect in nasopharyngeal sample (target: GAPDH) using cation charge particles

The results (Test 2) of performing RT-PCR after applying the DLB and the nucleic acid extraction method (Test 2; cation) using cation charge particles to a nasopharyngeal sample (nasopharyngeal cancer sample) and the results (control, Test 1) of performing RT-PCR after applying only the DLB to the same sample of the same amount were compared and illustrated using fluorescence amplification curves, and the results are shown in FIGS. 9 and 10.

As shown in the graph of FIG. 9, no fluorescence appeared in any of Test 1 and Test 2 (cation). Also, As shown in the graph of FIG. 10, no fluorescence appeared in any of Test 1 and Test 2 (cation).

From the results of Comparative Examples 1 and 2 above, it was confirmed that, unlike the present invention, when cationic porous ion charge particles were used, nucleic acids were not extracted or detected at all.

### Experimental Example 1. Nucleic acid isolation and detection test using various types of polymer absorbers

Nucleic acid isolation and detection tests were performed using various types of polymer absorbers, and the results are shown in FIG. 16.

Specifically, in the nucleic acid extraction method of the present invention, after applying only the DLB, applying cationic ion particles, applying negative fiber filter, and applying anion particle bead type, instead of the porous anion charge particles, PCR detection test were performed using solutions concentrated through each of the isolation methods (FIG. 16, 1. Using only DLB / 2. cation particles (0.1 g, 0.2 g, 0.3 g) / 3. Porous anion charge particles of the present invention (0.1 g, 0.2 g, 0.3g) / 4. Negative fiber filter (1 filter, 2 filters, 3 filters) / 5. Anion particle bead type (3 beads, 6 beads, 9 beads)).

As shown in FIG. 16, as a result of the PCR, it could be confirmed that when the porous anionic particles of the present invention were used, the nucleic acid isolation and detection efficacy was significantly better than when a negative fiber filter or an anion particle bead type was used.

## Claims

1. Porous ion charge particles for nucleic acid isolation.

2. The porous ion charge particles for nucleic acid isolation according to claim 1, wherein the porous ion charge particles are an anionic polyelectrolyte.

3. The porous ion charge particles for nucleic acid isolation according to claim 1, wherein the porous ion charge particles are one or more selected from the group consisting of polyacrylate, sodium polyacrylate, polyacrylamide, polyvinyl alcohol, polyethylene, polyacrylonitrile, alginate, ethylene maleic anhydride, methyl cellulose, hydroxy ethyl cellulose, hydroxypropyl methyl cellulose, cellulose acetate, carboxy methyl cellulose, and polymethacrylate (PMA).

4. The particles according to claim 3, wherein a material of the porous ion charge particles is sodium polyacrylate.

5. A nucleic acid extraction tube comprising the porous ion charge particles for nucleic acid isolation of any of claims 1 to 4.

6. A method of extracting nucleic acids, comprising:
adding and mixing a sample including nucleic acids into a nucleic acid extraction tube together with a direct lysis buffer;
adding porous ion charge particles to the resulting mixture and subjecting to a reaction for 5 to 30 minutes; and
removing the ion charge particles that have completed the reaction and obtaining a buffer solution in which nucleic acids are concentrated.

7. The method of extracting nucleic acids according to claim 6, wherein the porous ion charge particles are an anionic polyelectrolyte.

8. The method of extracting nucleic acids according to claim 7, wherein the porous ion charge particles are one or more selected from the group consisting of polyacrylate, sodium polyacrylate, polyacrylamide, polyvinyl alcohol, polyethylene, polyacrylonitrile, alginate, ethylene maleic anhydride, methyl cellulose, hydroxy ethyl cellulose, hydroxypropyl methyl cellulose, cellulose acetate, carboxy methyl cellulose, and polymethacrylate (PMA).

9. The method of extracting nucleic acids according to claim 8, wherein a material of the porous ion charge particles is sodium polyacrylate.

10. The method of extracting nucleic acids according to claim 6, wherein the porous ion charge particles adsorb a cationic by-product and absorb a solvent.

11. The method of extracting nucleic acids according to claim 6, wherein the reaction is performed at room temperature.

12. The method of extracting nucleic acids according to claim 6, wherein the reaction includes no further washing.

13. A method of extracting nucleic acids, comprising:
adding and mixing a sample including nucleic acids into a nucleic acid extraction tube together with a direct lysis buffer;
adding porous ion charge particles to the resulting mixture and subjecting to a reaction for 5 to 30 minutes;
removing the ion charge particles that have completed the reaction and obtaining a buffer solution in which nucleic acids are concentrated;
adding a polymerase chain reaction (PCR) mixed agent to the buffer solution in which nucleic acids are concentrated and performing a PCR; and
in-situ detecting a sample during the PCR or after completing the same.
